Europäisches Patentamt

European Patent Office

Office européen des brevets

(11) Numéro de publication : **0 368 363 B1**

(12) **FASCICULE DE BREVET EUROPEEN**

(45) Date de publication du fascicule du brevet :
**11.11.92 Bulletin 92/46**

(51) Int. Cl.$^5$ : **C07C 47/277**

(21) Numéro de dépôt : **89122806.6**

(22) Date de dépôt : **02.06.87**

(54) **Nouveau monoacétal du glyoxal.**

(30) Priorité : **03.06.86 FR 8607957**
**29.04.87 FR 8706106**

(43) Date de publication de la demande :
**16.05.90 Bulletin 90/20**

(45) Mention de la délivrance du brevet :
**11.11.92 Bulletin 92/46**

(84) Etats contractants désignés :
**AT BE CH DE ES FR GB GR IT LI LU NL SE**

(56) Documents cités :
**AT-B- 379 799**
**DE-A- 2 514 001**
**US-A- 2 116 016**
**JOURNAL OF ORGANICAL CHEMISTRY, vol. 38, no. 3, 1973, pages 556-560; J.M. KLIEGMAN et al.: "Glyoxal derivatives. V. Reactionof alcohols with glyoxal"**

(60) Numéro de publication de la demande initiale
en application de l'article 76 CBE : **0 249 530**

(73) Titulaire : **SOCIETE FRANCAISE HOECHST**
**TOUR ROUSSEL HOECHST 1 Terrasse Bellini**
**F-92800 Puteaux (FR)**

(72) Inventeur : **Blanc, Alain**
**21 bis rue Galvanis**
**F-75017 Paris (FR)**
Inventeur : **Hamedi-Sangsari, Farid**
**341 rue Piemente**
**F-69009 Lyon (FR)**
Inventeur : **Chastrette, Francine**
**22 chemin de la Combe Martin**
**F-69300 Caluire (FR)**

(74) Mandataire : **Rinuy, Santarelli**
**14, avenue de la Grande Armée**
**F-75017 Paris (FR)**

## Description

La présente invention concerne un nouveau monoacétal du glyoxal et son application comme synthon d'oléfinoformylation.

Les réactions du glyoxal avec les alcools ont été très étudiées et elles permettent d'accéder facilement au glyoxal bisacétal correspondant (brevet US N° 2 194 405; brevet GB N° 559 362 ; brevets français N° 1 280 792 et 2 284 584 ; H. Fischer et al, Chem. Ber., 1926, 59, 851 ; D.H. Grangaard et al, J. Amer. Chem. Soc. 1939, 61, 428 et 755 ; M. Sprung et al, J. Amer. Chem. Soc., 1951, 73, 1884 ; H. Fiesselmann et al, Chem. Ber., 1954, 87, 906 ; U. Faas et al, Chem. Ber., 1954, 87, 1343; J.M. Kliegman et al, J. Org. Chem., 1972, 37, 1276 ; ibid 1973, 38, 556 ; ibid 1974, 39, 1172 ; F. Chastrette et al, Bull. Soc. Chim. France, 1976, 601 et 613).

Ces méthodes directes n'ont pas permis d'accéder aux monoacétals du glyoxal, si bien que, pour les obtenir, on a eu recours à des méthodes indirectes telles que l'ozonolyse de l'acétal correspondant de l'acroléine ou la coupure oxydante d'un acétal $\alpha,\beta$-dihydroxylé comme le dihydroxy-2,3-tétraéthoxy-1,1,4,4 butane (C. Harries, Chem. Ber., 1903, 36, 1933 ; H. Fischer et al, Helv. Chim. Acta., 1939, 18, 514 ; L. Yanovkaya et al, Izvest. Akad. Nauk SSR, Otdel Khim. Nauk, 1963, 857 ; J. Hine et al, J. Amer. Chem. Soc., 1972, 94, 6998 ; P. Noire, Chemical Abstracts, 1978, 89, référence 215108).

Ces procédés sont longs, onéreux et même parfois dangereux lorsqu'ils sont mis en oeuvre sur des quantités importantes (cf. brevet de la R.F.A. N° 3 346 266).

Or la Demanderesse a découvert avec étonnement un procédé pour accéder rapidement et avec de bons rendements à un nouveau monoacétal du glyoxal.

C'est pourquoi la présente demande a pour objet le diallyloxy-2,2-éthanal. Ce dernier peut être préparé par un procédé qui consiste à faire réagir du glyoxal en présence d'un catalyseur acide avec un excès d'alcool allylique puis à arrêter la réaction dès que la concentration en monoacétal cherché décroît dans le milieu réactionnel au profit du bisacétal : le tétraallyloxy-1,1,2,2-éthane. La Demanderesse s'est en effet aperçue que le glyoxal, même en solution aqueuse, réagissait rapidement avec l'alcool ci-dessus pour donner le monoacétal correspondant qui se transformait ensuite moins rapidement en bisacétal.

Dans les très nombreux travaux consacrés à la condensation des alcools avec le glyoxal, jamais cette dissociation dans la formation du mono- et du bisacétal du glyoxal n'avait été prévue, entrevue, signalée ou obtenue. Si bien que la simplicité du procédé ci-dessus permet d'accéder aisément au diallyloxy-2,2 éthanal, qui ne peut être préparé par les méthodes connues.

Selon le procédé ci-dessus, on suit d'une part la disparition du glyoxal et d'autre part, la formation des acétals par l'analyse d'échantillons prélevés régulièrement dans le milieu réactionnel.

La consommation du glyoxal est suivie, par exemple, par détermination de la soude nécessaire à sa transformation en glycolate de sodium, selon la réaction de Cannizzaro. Quant aux mono- et bisacétals, ils sont dosés avantageusement par chromatographie en phase gazeuse après détermination des coefficients de réponse par la méthode classique d'étalonnage interne.

Comme toutes les réactions d'acétalisation, le procédé ci-dessus est effectué en présence d'un catalyseur acide. Parmi ces catalyseurs acides, on peut citer le chlorure d'hydrogène, l'acide sulfurique, l'acide paratoluènesulfonique, le sulfate de zirconium (IV), les résines sulfoniques échangeuses d'ions sous forme acide. On emploie habituellement $50 \pm 25$ mmoles du catalyseur acide par mole de glyoxal engagée. En fin de réaction, ce catalyseur acide est rapidement éliminé du milieu réactionnel par des moyens connus en eux-mêmes, comme par filtration, neutralisation au moyen d'une base appropriée, etc.

La réaction est mise en oeuvre avec un excès d'alcool par rapport au glyoxal. Cet excès peut varier dans de grandes proportions mais habituellement on emploie $12 \pm 5$ moles d'alcool par mole de glyoxal. Si nécessaire, on peut opérer au sein d'un solvant organique inerte compatible avec ce type de réaction de substitution nucléophile acido-catalysée tel que l'hexane, le cyclohexane, le benzène, le toluène, les solvants chlorés : chloroforme, dichlorométhane.

Le glyoxal de départ est soit solide, comme son trimère cristallisé avec deux molécules d'eau, soit en solution aqueuse. Dans ce dernier cas, on peut éliminer une partie de l'eau de dissolution par distillation azéotropique préalable.

On conduit habituellement la réaction à la température d'ébullition du milieu réactionnel mais on peut la conduire à des températures supérieures ou inférieures à celle-ci. On travaille habituellement avec distillation azéotropique de l'eau présente et/ou formée dans le milieu réactionnel avec recyclage du distillat déshydraté par des moyens connus en eux-mêmes, tels que la lixiviation à travers une substance desséchante comme le sulfate de magnésium anhydre.

La réaction est généralement conduite à pression atmosphérique bien qu'une pression supérieure ou inférieure ne soient pas nuisibles au procédé.

En fin de réaction, le monoacétal du glyoxal cherché est isolé du milieu réactionnel par des moyens connus en eux-mêmes tels que la distillation fractionnée ou la cristallisation.

Le diallyloxy-2,2-éthanal est un synthon d'oléfinoformylation très intéressant en synthèse organique. Il peut être, comme d'autres monoacétals du glyoxal, employé pour accéder notamment à certains hétérocycles (D. Soerens et al, J. Org. Chem. 1979, 44, 535).

C'est pourquoi la présente demande a également pour objet l'application du diallyloxy-2,2-éthanal comme synthon d'oléfinoformylation.

L'exemple suivant illustre l'invention sans toutefois la limiter.

Exemple : Préparation du diallyloxy-2,2-éthanal

On chauffe au reflux pendant t heures avec distillation azéotropique de l'eau et recyclage du solvant organique, à l'aide d'un appareil Dean Stark, une solution de :
- 1 mole de glyoxal en solution aqueuse à 40 % en poids,
- 50 mmoles d'un catalyseur C (ou 50 méq. acide d'une résine sulfonique),
- 10 moles d'alcool allylique,
- 1600 cm$^3$ d'un solvant S donnant un azéotrope avec l'eau.

Lorsque les dosages de diallyloxy-2,2-éthanal et de tétraallyloxy-1,1,2,2 éthane effectués périodiquement au cours du chauffage indiquent une décroissance de la concentration en diallyloxy-2,2 éthanal au profit du tétraallyloxy-1,1,2,2 éthane, on refroidit le milieu réactionnel puis on élimine le catalyseur par filtration et/ou par neutralisation avec un carbonate acide d'un métal alcalin suivie d'une filtration et ensuite on distille le milieu réactionnel. Après élimination des solvants, on recueille une première fraction distillant à $62 \pm 2°C$ sous 6 mbar de p moles constituée par le produit attendu puis une deuxième fraction distillant à $100 \pm 5°C$ sous 5 mbar de q moles constituée par le tétraallyloxy-1,2,2,2 éthane.

Le tableau I ci-après résume les essais effectués.

## Tableau I

| Essai N° | t en heures | C catalyseur | S solvant | p | q |
|---|---|---|---|---|---|
| 1 | 2 | $H_2SO_4$ conc | benzène | 0,49 | 0,18 |
| 2 | 2 | $Zr(SO_4)_2$ | benzène | 0,51 | 0,27 |
| 3 | 5 | Résine A | benzène | 0,70 | 0,16 |
| 4 | 1,5 | Résine B | benzène | 0,54 | 0,29 |
| 5 | 5 | $Zr(SO_4)_2$ | chloroforme | 0,53 | 0,12 |
| 6 | 10 | Résine A | chloroforme | 0,70 | 0,13 |
| 7 | 6 | Résine B | chloroforme | 0,57 | 0,23 |

Résine A : résine macropore commercialisée sous la dénomination IMAC C16 P (cf. Encyclopedia of Chemical Technology, Kirk-Othmer, 3ème édition, 13, 696).

Résine B : résine NAFION (marque enregistrée) (cf. Encyclopedia of Chemical Technology, Kirk-Othmer, 3ème édition S 559).

Analyses physiques

RMN$^1$H (CDCl$_3$)
4,2 ppm (doublet, 4H, -OCH$_2$)
4,7 ppm (doublet, 1H, J = 2 Hz, > CH-C)
5,2 ppm (massif, 4H, CH$_2$)
5,8 ppm (massif, 2H, =CH-)
9,5 ppm (doublet, 1H, J = 2 Hz, CHO)
A la connaissance de la Demanderesse, ce produit n'est pas décrit dans la littérature.

**Revendications**

1.   Le diallyloxy-2,2 éthanal.

2.   Application du diallyloxy-2,2 éthanal à titre de synthon d'oléfinoformylation.

**Patentansprüche**

1.   Diallyloxy-2,2-ethanal

2.   Verwendung von Diallyloxy-2,2-ethanal als Synthon der Olefinformylierung.

**Claims**

1.   2,2-diallyloxy ethanal.

2.   Use of 2,2-diallyloxy ethanal as an olefin formylation synthon.